# EUROPÄISCHE PATENTANMELDUNG

(11) **EP 4 154 894 A1**
(43) Veröffentlichungstag der Anmeldung: **29.03.2023**
(21) Anmeldenummer: 22197360.5
(22) Anmeldetag: 23.09.2022
(51) Int. Cl.: A61K 35/16, A61K 35/14, A61K 35/18, A61K 35/19, C12N 5/078, A61P 17/02, A61P 19/02, A61P 37/06

(54) **AUTOLOGES THERAPEUTIKUM UND VERFAHREN ZUR HERSTELLUNG DESSELBEN**

(30) Priorität: 24.09.2021 DE 102021124752
(71) Anmelder: Ad Lentus GmbH, 89077 Ulm (DE)
(72) Erfinder: Ludwig, Cornelia Maria, 89188 Merklingen, DE (DE); Steiger, Jörn, 89077 Ulm (DE)
(74) Vertreter: Meyer, Thorsten

(57) **Zusammenfassung**

Die Erfindung betrifft ein Verfahren zur Herstellung eines mit Faktoren und Botenstoffen angereichertem autologen Therapeutikums aus einer Blutspende, mit den Schritten:
- Aufteilen der Menge C des Bluts eines Blutspenders (1) zu zwei Teilen A und B,
- Inkubation des Teils A für einen vorgewählten Zeitraum in einem Brutschrank durch thermische Beaufschlagung bei 35° bis 40°C, insbesondere bei 37°C,
- Abtrennung der Fraktion der Leukozyten durch Isolierung aus Teil
B und Beimengung von einem isotonischen Puffer (NaCl) zu Teil D;
- Inkubation des Teils D unter Nährstoffmangel und/oder Sauerstoffmangel im Verhältnis zum normalen Bedarf der Zellen;
- Zusammenführen der Teile A und D zu einer Mischung E

Des Weiteren betrifft die Erfindung ein autologes Therapeutikum, eine pharmazeutische Zusammensetzung und eine Verwendung derselben bei einer Therapie.

## Beschreibung

Die vorliegende Erfindung betrifft ein zellfreies autologes Immuntherapeutikum und ein Verfahren zur Herstellung bzw. Gewinnung eines mit Wachstumsfaktoren und Botenstoffen (Zytokinen) angereicherten Blutplasmagemisches.

Dieses Therapeutikum kann für die Vorbeugung oder Behandlung von Erkrankungen, die mit einer überschießenden Reaktion des Immunsystems einhergehen, eingesetzt werden. Eine überschießende Immunreaktion kann beispielsweise eine Entzündungsreaktion darstellen, die mit einem Zytokinsturm (übermäßige Produktion von Zytokinen) verbunden ist, die eine Zerstörung des damit verbundenen Gewebes und Organschädigungen zur Folge haben können.

Das Verfahren beinhaltet eine Inkubation des Vollblutes und der aus dem Blut gewonnenen Leukozyten unter Stressbedingungen. Diese Stressbedingungen umfassen Sauerstoff- und Nährstoffmangel, sowie Temperaturstress. Die Inkubation der Zellen unter Mangelbedingungen für einen bestimmten Zeitraum führt zur Sekretion zellulärer Botenstoffe, wie Wachstumsfaktoren und Zytokine, ins Umgebungsmedium. Dieses Medium kann im vorliegenden Verfahren als konditioniertes Medium bezeichnet werden. Im Anschluss an dieses Verfahren kann die so gewonnene Plasmasuspension wieder zurück in den Patienten gegeben werden, wobei die nun erzeugten Botenstoffe einen regulatorischen (immunmodulatorischen) Einfluss auf das Immunsystem des Patienten erzielen können. Diese Immunmodulation kann beispielsweise anti-inflammatorisch wirken. Unter anderem kann sie bei Patienten mit einer akuten Entzündungsreaktion (Inflammation), bzw. Zytokinsturm-assoziierte Reaktionen des Immunsystems eine Besserung des physischen Zustandes hervorrufen oder beispielsweise ein akutes Organversagen verhindern.

Bestimmte Erkrankungen gehen mit einer chronischen überschießenden Reaktion des Immunsystems des Patienten einher, bei denen zelluläre Botenstoffe wie Zytokine in erhöhter Menge produziert werden und damit zu einer chronischen Entzündung und sogar Gewebszerstörung führen können. Beispielsweise tritt bei der rheumatoiden Arthritis ein erhöhter Spiegel an Tumor Nekrose Faktor Alpha (TNF-α) auf, der zu einer chronischen Zerstörung der Gelenke führen kann.

Außerdem gibt es akute Erkrankungen, die mit einer überschießenden Immunreaktion einhergehen. Beispielsweise bei der neuartigen COVID-19 Erkrankung, die durch das SARS-CoV-2 Virus ausgelöst wird, kann es zu einer Überreaktion des Immunsystems infolge der Virusbekämpfung kommen, die sich dann z.B. gegen das Lungengewebe richten kann. Bei dieser Überreaktion kommt es zu einem erhöhten Spiegel an Botenstoffen wie Zytokinen. Diese von Zellen des Immunsystems gebildeten und sezernierten Faktoren können eine starke Entzündungsreaktion verursachen. Bei COVID-19 Patienten wurden hohe Spiegel an pro-inflammatorischen Zytokinen, also entzündungsfördernde Botenstoffe, wie beispielsweise GCSF, IP10, MCP1, MIP1A und TNFα gemessen (Entzündungsreaktion verursachen et al., Immunity, 2020). Dieser Zytokinsturm kann zu einem akuten respiratorischen Distress Syndrome (ARDS) führen und in der Folge zur Zerstörung des Lungengewebes. Bisher gibt es noch wenig Therapiemöglichkeiten zur Behandlung solcher schwerwiegender Entzündungsreaktionen.

Bei manchen Erkrankungen, die eine verbesserte Wundheilung erfordern, wird eine Eigenbluttherapie oder ein PRP Verfahren (Plättchen reiches Plasma, d.h. mit Thrombozyten angereichertes Plasma oder auch autologes konditioniertes Plasma genannt) angewendet, welches allerdings nicht standardisiert eingesetzt werden kann und keine gezielte Stimulation von Zellen im Prozess beinhaltet.

Neuartige und vielversprechende Therapieansätze, die einen immunmodulatorischen Weg verfolgen, bieten somatische Zelltherapien wie z.B. der Einsatz von Mesenchymalen Stammzellen (MSC) und ihre Sekretionsprodukte, die Exosmosen (Gupta et. al, Human Cell, 2020). MSC sind heterogene Zellen, die immunmodulatorische und entzündungshemmende Eigenschaften besitzen, indem sie anti-inflammatorische Wachstumsfaktoren und Zytokine sezernieren. Ein Vorteil dieser Therapien besteht darin, dass körpereigene Zellen (d.h. autologes Verfahren) und damit auch deren modulierende/ regulierende Botenstoffe zum Einsatz kommen und daher spezifischer wirken als klassische Arzneimittel, und somit keine schweren Nebenwirkungen zu erwarten sind. Mithilfe der MSC Therapie können gleichzeitig auch mehrere Zytokine und Wachstumsfaktoren reguliert werden, was bei einer Arzneimittel- oder gezielten Antikörpertherapie nicht erreicht werden kann.

Allerdings werden bei der MSC-Therapie Stammzellen aus dem Knochenmark des Patienten entnommen, welches einen schmerzhaften operativen Eingriff für den Patienten darstellt. Alternativ könnte man die Zellen zwar auch aus anderem Gewebe, wie z.B. dem Fettgewebe isolieren, allerdings ist hier ebenfalls ein operativer Eingriff notwendig. Ein weiterer Nachteil besteht darin, dass die MSCs zunächst kultiviert, d.h. passagiert und expandiert (vermehrt) werden müssen, da sie nur in sehr geringer Anzahl im Körper vorhanden sind. Dies geht mit einem hohen Zeitaufwand von mindestens 14 Tagen einher. Diese Zeit geht für den Patienten, dem in einer akuten Situation möglichst schnell geholfen werden muss, verloren. Außerdem besteht die Gefahr von Kontaminationen, die größer wird, je länger die Zellen in Kultur sind.

Darüber hinaus kann es beispielsweise bei einem mit SARS-CoV-2 infizierten Patienten durch die notwendigen hohen Sicherheitsanforderungen aufwendiger und umständlicher sein, diese Mesenchymalen Stammzellen zu kultivieren, da die damit verbundene Laborausrüstung unter diese Sicherheitsmaßnahmen fallen würden.

Eine Unsicherheit besteht auch in dem Verhalten bzw. Verweildauer dieser MSCs im Körper. Es wurde beispielsweise berichtet, dass intravenös verabreichte MSCs schnell in der Lunge eingefangen werden und sich nicht weiter im Körper verteilen können. Hilfreicher wäre es, nur die immunmodulierenden Botenstoffe (Zytokine und Wachstumsfaktoren) einsetzen zu können, die dann nach Behandlung auch wieder vom Körper selbst abgebaut werden können und die Behandlung somit berechenbarer und nebenwirkungsärmer machen. Es wurden auch die in den Überständen der Zellkulturen enthaltenen Exosomen verwendet. Exosomen können Vesikel oder Bläschen darstellen, die von einer einfachen oder doppelten Membran oder netzartigen Hülle aus Proteinen umgeben sind, und die von Zellen in die Umgebung abgegeben werden. Exosomen zeigen ebenfalls immunmodulatorische Eigenschaften, doch hier besteht ebenfalls der Nachteil, dass eine aufwendige Zellkultur bzw. Aufreinigung der Exosomen, erforderlich ist und man immer noch zusätzlich vorher MSCs gewinnen und kultivieren muss.

Daher besteht ein der vorliegenden Erfindung zugrundeliegendes Problem darin, ein Verfahren bereitzustellen, welches mit wenig Zeit, Aufwand und Kontaminationsrisiko verbunden ist, und bei dem ein immunmodulatorisch wirksames autolog hergestelltes Präparat generiert werden kann, welches Botenstoffe (wie Wachstumsfaktoren, Zytokine) enthält. Beispielsweise könnten Patienten in einer Akutsituation daher zeitnah therapiert werden. Außerdem sollte dieses Verfahren zur Gewinnung eines immunmodulierend wirksamen Therapeutikums (Immuntherapeutikum), mit wenig Schmerzen und geringem operativen Aufwand für den Patienten verbunden sein. Dies wäre beispielsweise bei chronisch erkrankten Patienten von Vorteil, wenn eine Therapie wiederholt erfolgen soll.

Die oben genannten Aufgaben werden durch Anwendung eines Verfahrens nach Anspruch 1 gelöst.

Bei der Behandlung von akut erkrankten Patienten (beispielsweise COVID-19) besteht die Notwendigkeit ein schnelles Verfahren mit geringem Aufwand und wenig aufwendigem Labormaterial bereitzustellen, um den Patienten in jeder klinischen Einrichtung, zu helfen, ohne dafür eine weitere spezielle Ausrüstung zu benötigen.

Dies wird durch die vorliegende Erfindung dadurch gelöst, dass es ein Verfahren ist, welches mit der Standard Laborausrüstung durchgeführt werden kann, also in jeder behandelnden Einheit, die eine Blutabnahme durchführen kann. Es werden für das Verfahren Röhrchen, Pipetten und Zentrifugen, sowie eine mikrobiologische Sicherheitswerkbank benötigt.

Genauer offenbart das Verfahren nach der Erfindung zur Herstellung eines mit Faktoren und Botenstoffen angereichertem autologen Therapeutikums aus einer Blutspende die Schritte:
- Aufteilen der Menge C des Blutes eines Blutspenders zu zwei Teilen A und B,
- Inkubation des Teils A für einen vorgewählten Zeitraum durch thermische Beaufschlagung bei 35° bis 40°C, insbesondere bei 37°C,
- Abtrennung der Fraktion der Leukozyten durch Isolierung aus Teil
B und Beimengung von einem isotonischen Puffer (NaCl) zu Teil D;
- Inkubation des Teils D unter Nährstoffmangel und/oder Sauerstoffmangel im Verhältnis zum normalen Bedarf der Zellen;
- Zusammenführen der Teile A und D zu einer Mischung E.

In einem weiteren Schritt werden aus der Mischung E die Zellen abfiltriert.

Ein Detail sieht vor, dass die Menge C des Blutes vor dem Aufteilen mit einem Anti-Koagulans versetzt wird, vorzugsweise Heparin oder Citrat Puffer.

Von Vorteil ist, dass das Blut direkt nach der Blutabnahme mit einer Temperatur unterhalb der normalen Körpertemperatur des Blutspenders über einen vorgewählten Zeitraum von mehreren Stunden gelagert werden kann.

Der Teil A des Blutes wird vorteilhafterweise nach der Inkubation zur Trennung des Plasmas von den restlichen Bestandteilen des Blutes abzentrifugiert, wobei die restlichen Bestandteile entfernt werden.

Die Abtrennung der Fraktion der Leukozyten durch Isolierung aus dem Teil B (PBS/Blut-Gemisch) erfolgt bevorzugterweise mittels Dichtegradientenzentrifugation oder magnetic bead sorting.

Gemäß einer Ausgestaltung des Verfahrens ist vorgesehen, dass die Inkubation (thermische Stimulation) der isolierten Leukozyten aus Teil B für 2 bis 8 Stunden in einem isotonen Puffer, vorzugsweise 0,9% NaCl, aq bei 35 bis 40°C, bevorzugt bei 37°C, erfolgt.

Vorteilhaft ist, dass das Plasma des inkubierten Teils A, insbesondere nach Zentrifugation, bei -20 bis -180°C kryogelagert wird, vorzugsweise bei einer Temperatur von -150°C und vor der Herstellung des applikationsfertigen Endproduktes aufgetaut wird.

Nach einem weiteren Aspekt der Erfindung ist ein autologes Therapeutikum nach einem der Ansprüche 1 bis 8 zur Verwendung als Medikament vorgeschlagen.

Ein weiterer Aspekt sieht ein autologes Therapeutikum nach Anspruch 9 zur Verwendung bei der Behandlung von einer der nachfolgenden Erkrankungen vor:
- rheumatoider Arthritis, Arthritis,
- Inflammationen (Entzündungen),
- Wundheilungsverzug, Wundheilungsstörungen,
- chronische Wunden, insbesondere Ulkus oder anal Fisteln,
- Frostbeulen, Gefäßverengungen,
- COVID-19 - SARS induzierte Entzündungsreaktionen,
- Lungenbluten (Pferde).

Pharmazeutische Zusammensetzung ist nach einem weiteren Aspekt vorgeschlagen, umfassend ein autologes Therapeutikum nach einem der Ansprüche 9 bis 10 und einen oder mehreren pharmazeutisch annehmbaren Hilfsstoffen.

Nach einem weiteren Aspekt der Erfindung ist vorgeschlagen: ein autologes Therapeutikum nach einem der Ansprüche 9 bis 10 oder eine pharmazeutische Zusammensetzung nach Anspruch 11 zur Verwendung bei einer Therapie, oder in einem Verfahren zum Behandeln einer Krankheit oder eines Zustands, wobei das Verfahren ein Verabreichen einer therapeutisch wirksamen Menge des Therapeutikums an ein Subjekt umfasst, wobei die Krankheit oder Zustand ein Entzündungszustand, eine immunregulatorische Störung ist.

Anschließend ist nach einem weiteren Aspekt ein autologes Therapeutikum nach Anspruch 9 vorgesehen, wobei die Anwendung als Medikament subkutan oder intradermal erfolgt.

Weitere vorteilhafte Ausgestaltungen ergeben sich aus den weiteren Unteransprüchen oder deren mögliche Unterkombinationen.

Dieses der Erfindung zugrundeliegende Verfahren kann alternativ zu autolog auch als extrakorporale Behandlung der Leukozyten bezeichnet werden, das heißt, die Zellen werden außerhalb des Körpers behandelt, wobei mithilfe gezielter Stimulation die Leukozyten zur Sekretion von Botenstoffen, wie Wachstumsfaktoren und Zytokinen, angeregt werden.

Bei dem vorliegenden Verfahren wird ex vivo (außerhalb des menschlichen oder tierischen Körpers) ein autologes immunologisch wirksames Therapeutikum gewonnen. Aus venös entnommenem Blut werden, mithilfe dieses Prozesses, Wachstumsfaktoren und Zytokine (u.a. Chemokine), im Blutplasma erzeugt, die einen immunmodulierenden Einfluss beispielsweise haben können. Dieser Einfluss kann vorzugsweise, aber nicht ausschließlich, anti-entzündlich sein.

In einem ersten Schritt des vorliegenden Verfahrens wird, nach Blutentnahme des Patienten (Blutspender), das Vollblut in einer Menge von 50 - 500 ml mit einem Anti-Koagulanz zusammengebracht. Das Vollblut befindet sich vorzugsweise in Spritzen oder Monovetten. Das Anti-Koagulanz kann vorzugsweise, aber nicht ausschließlich Heparin oder Citratpuffer sein. In einer weiteren Ausführung kann das Röhrchen der Spritzen oder Monovetten mit einem Anti-Koagulanz, wie beispielsweise Heparin beschichtet sein. Unter Anti-Koagulantien werden Stoffe, Verbindungen bezeichnet, die in der Lage sind die Gerinnung des Blutes zu verhindern.

Die für das Verfahren verwendete Behälter oder Gefäße können vorzugsweise 50 ml Zentrifugenröhrchen sein.

In jedem Schritt des vorliegenden Verfahrens befindet sich das Blut in einem geschlossenen System, vorzugsweise Spritzen oder Monovetten, alternativ auch in einem Vaccutainer. Ein geschlossenes System bedeutet erfindungsgemäß, dass kein zusätzlicher Sauerstoff oder Kohlenstoffdioxid in das Gefäß und somit zu den Zellen, gelangt. Dies gewährleistet den Stresszustand der Zellen und veranlasst somit die Sekretion der Faktoren und Botenstoffe.

In einem nächsten Schritt kann das Vollblut transportiert oder gelagert werden. Die Temperatur während dieses Vorganges beträgt zwischen 18 - 24 °C, vorzugsweise 20°C. Die Transport- bzw. Lagerungszeit beträgt über Nacht, vorzugsweise 12-18 Stunden (h).

Dieser Schritt beinhaltet bereits eine Sauerstoffmangelversorgung der Zellen aufgrund des verschlossenen Gefäßes, wobei es hier zu einer Sekretion der zellulären Botenstoffe kommen kann.

In einem nächsten Schritt wird in einem Labor das gesamte Blut zu einer Menge C gepoolt, das heißt zusammengeführt und anschließend in zwei gleiche Volumen Teile A und B in Röhrchen aufgeteilt.

Teil A wird im Weiteren in einem Brutschrank (vorzugsweise Zellkultur-Brutschrank) über Nacht unter thermischer Beaufschlagung bei 35° bis 40°, insbesondere bei 37°, inkubiert. Die Inkubationszeit beträgt hierbei 16 bis 26 Stunden, vorzugsweise 24 Stunden. Im Anschluss wird das Blut erneut in einem Gefäß gesammelt (gepoolt). Nach der Inkubation wird das Blutplasma von den übrigen Bestandteilen des Blutes durch Zentrifugation abgetrennt und kann beispielsweise kryogelagert werden, bis es mit dem im folgenden als Teil D beschriebenem Gemisch zusammengebracht wird. Das Plasma ist nun mit beispielsweise Wachstumsfaktoren und Botenstoffen (z.B. Zytokine, Interleukine und Mikrovesikel) angereichert, kann daher auch als konditioniertes Medium oder aktiviertes Plasma bezeichnet werden. Als konditioniertes Medium (conditioned medium, CM) wird im Allgemeinen ein Medium bezeichnet, welches die Faktoren enthält, die der jeweilige Zelltyp sezerniert hat. Es kann zur weiteren Kultur anderer Zellen verwendet werden, die auf diese Faktoren angewiesen sind. Gemäß dieser Erfindung wird als konditionertes Medium das mit Wachstumsfaktoren und Botenstoffen angereicherte Blutplasma bezeichnet.

Das Verfahren beinhaltet ferner eine Leukozytenstimulation. Hierbei erfolgt die Abtrennung der Leukozyten-Fraktion durch Isolierung aus Teil B. In einem ersten Schritt erfolgt eine Beimengung von einem isotonischen Puffer, vorzugsweise NaCl, insbesondere 0,9% ige NaCl Lösung, zu Teil B. Das nun entstandene Gemisch stellt Teil D dar. Alternativ kann auch PBS (Phosphate Buffered Saline) ohne weitere Salze wie Calcium (Ca++) oder Magnesium (Mg++) als isotonischer Puffer verwendet werden. Das Mischungsverhältnis von Teil B (Leukozyten) und isotonen Puffer beträgt vorzugsweise 1:2, kann aber auch 1:3 oder 1:4 betragen. In einem zweiten Schritt werden die Leukozyten vom Plasma abgetrennt. Dies kann vorzugsweise mittels einer Dichtegradientenzentrifugation bei 600 g bis 900 g, vorzugsweise 800 g oder mithilfe eines magnetic bead sorters durchgeführt werden.

Das Verfahren beinhaltet im Weiteren die Inkubation von Teil D, also dem Leukozyten-Puffergemisch, unter Nährstoff- und Sauerstoffmangel im Verhältnis zum normalen Bedarf der Zellen. Das bedeutet die Leukozyten werden einer Stresssituation ausgesetzt und dadurch zur Sekretion von Botenstoffen angeregt. Das vorliegende Verfahren beinhaltet also eine Induktion von anti-inflammatorischen und immunmodulatorischen Faktoren (z.B. Wachstumsfaktoren) und Botenstoffen und löst damit ein weiteres Problem der schnellen Bereitstellung eines autologen Therapeutikums mit anti-inflammatorischen Faktoren für die Behandlung von Patienten mit einer Inflammation, also einer Entzündungsreaktion.

Der Nährstoffmangel wird in der vorliegenden Erfindung durch die Inkubation mit 0,9 %iger NaCl Lösung erreicht. Daher erfolgt die Inkubation in Abwesenheit sämtlicher Nährstoffe, die die Zellen zum Überleben benötigen, wie z.B. von Salz Ionen, vorzugsweise Ca²⁺ und Mg²⁺. In einer alternativen Ausführung kann aber auch PBS (Phosphate buffered Saline) verwendet werden.

Der Sauerstoffmangel oder die verminderte Sauerstoffzufuhr werden im vorliegenden Verfahren durch Inkubation der Zellen in fest verschlossenen Gefäßen (Monovetten oder Zentrifugenröhrchen) unter normalem Umgebungsdruck im Brutschrank erzeugt.

Die Inkubationszeit des Teils D unter Sauerstoff- und/oder Nährstoffmangel kann 2-8 Stunden, vorzugsweise 4 Stunden betragen, wobei die Temperatur bei 18° bis 24°C, bevorzugt bei 20°C gewählt wird.

Das Verfahren beinhaltet im Anschluss das Zusammenführen von Teil A und Teil D zu einer Menge E. Das Mischungsverhältnis kann beispielsweise zwischen 70 und 95% Anteil Teil D und entsprechend 5 bis 30% Anteil Teil A, insbesondere 80% Teil D und 20% Teil A, betragen. Alternativ kann das Mischungsverhältnis der Teile (oder Teilmengen) auch umgekehrt sein, also zwischen 70 und 95% Anteil Teil A und entsprechend 5 bis 30% Anteil Teil D.

Das wesentliche im vorliegenden Verfahren ist eine Generierung von Teilen (bzw. Teilmengen) des abgenommenen Blutes und die unterschiedliche Behandlung (Stressbedingungen wie Nährstoff-, Sauerstoff- und/oder Temperaturmangel) dieser Teile bzw. Teilmengen mit anschließender Zusammenführung bzw. Mischung der Teile zu einem wirksamen Immuntherapeutikum.

Das Zusammenführen kann in einer Monovette, Röhrchen oder Spritze erfolgen unter Raumtemperatur, vorzugsweise bei 20°C.

Teil A des vorliegenden Verfahrens kann als autologes oder aktiviertes Blutplasma bezeichnet werden, da die Zellen unter Sauerstoffmangel gestellt werden. Teil D wird in dem zugrundeliegenden Verfahren als aktivierte, d.h. unter Stress gestellte Leukozytenfraktion in einem isotonischen Puffer, bezeichnet. Nach Zusammenführen wird das Gemisch (Menge E) durch Sterilfiltration bzw. Spritzenfiltration mit einer Porengröße von 0.2µm - 5.0 µm, vorzugsweise 0.22 µm, 0.7 µm oder 0.37µm, insbesondere 0,45 µm sterilisiert. Dieser Vorgang entfernt die darin enthaltenen Zellen (Leukozyten, Thrombozyten) bzw. Zelltrümmer/Bestandteile. Antikörper oder andere Faktoren wie z.B. Blutgerinnungsfaktoren bleiben beispielsweise ebenfalls enthalten. Diese fertige Mischung kann als zellfreies, autologes Plasmagemisch bezeichnet oder als somatisches Immuntherapeutikum bezeichnet werden, welches unter anderem anti-entzündliche Eigenschaften besitzt.

Das durch das Verfahren gewonnene Immuntherapeutikum kann sofort dem Patienten appliziert werden. Dem Arzt kann praktischerweise eine kleine Box bereitgestellt werden, in der sich z.B. zwei 1 ml Spritzen befinden. Jede dieser Spritzen ist mit 500µl des erfindungsgemäß hergestellten Immuntherapeutikums gefüllt und mit Nadeln versehen, sodass eine sofortige Anwendung erfolgen kann. Das somatische Therapeutikum kann vorzugsweise innerhalb 72 Stunden nach Herstellung verwendet werden und kann bei 4°C - 10°C bis zu 24 Stunden aufbewahrt werden.

Durch das Verfahren wird ein autologes Blutplasmapräparat mit konditioniertem, d.h. mit zellulären Botenstoffen angereichertem Medium hergestellt. Unter aktiviertem Blutplasma versteht man in der vorliegenden Erfindung das Blutplasma, welches Leukozyten enthält, die unter Stressbedingungen kultiviert werden. Das gesamte Plasma wird bei einer Temperatur von 35°C - 40°C, vorzugsweise 37°C inkubiert. Leukozyten sind Bestandteile der weißen Blutkörperchen. Sie werden in der Thymusdrüse und dem Knochenmark gebildet und gelangen durch den Blut- und den Lymphstrom in alle Regionen des Körpers. Es gibt unterschiedliche Leukozyten-Typen, die jeweils eigene Aufgaben bei der Immunabwehr wahrnehmen (z.B. B-Lymphozyten, T-Lymphozyten, Dendritische Zellen, Granulozyten, Makrophagen). Jede dieser Zelltypen kann unterschiedliche Botenstoffe sezernieren. Somit können viele verschiedene Wachstumsfaktoren und Zytokine durch das der Erfindung zugrundeliegendes Verfahren generiert werden. Daher weist das, durch das Verfahren bereitgestellte Immuntherapeutikum ein breites Wirkungsspektrum auf. Insbesondere, aber nicht ausschließlich, können im vorliegenden Verfahren durch Th2 Helferzellen folgende Botenstoffe, genannt Interleukine (IL) generiert werden: IL-3, IL-4, IL-5, IL-6, IL-10, IL-13, IL-17E (IL-25), IL-31 die dann einen anti-inflammatorischen und immunmodulatorischen Einfluss nach Applikation in den Patienten haben können. Außerdem können auch Chemokine wie z.B. CXCL-8, CCL2, CCL3, CCL4, CCL5, CCL11, CXCL10 vorhanden sein. Ein Anteil an pro-inflammatorischen Zytokinen wie z.B. In, IL1 β, TNFα kann auch in der Präparation vorhanden sein. Ebenso können auch Wachstumsfaktoren wie z.B. Platelet derived growth factor, transforming growth factor β1 und β2, EGF (epidermaler Wachstumsfaktor) Fibroblast growth factor, epithelial growth factor, insulinähnliche Wachstumsfaktoren" und "Platelet-Derived Angiogenesis Factor" vorhanden sein.

Neben Zytokinen und Wachstumsfaktoren können beispielsweise auch Antikörper, Blutgerinnungsfaktoren und Mikrovesikel bzw. Exosomen sowohl im Blutplasma (Teil A) als auch im Teil D, dem Leukozyten Medium (isotonischer Puffer), vorhanden sein, die einzeln oder in Kombination zu einer Immunmodulation (Einfluss auf das Immunsystem) führen können.

Das in der vorliegenden Erfindung beschriebene Verfahren verwendet eine sogenannte Langzeitstimulation von vorzugsweise insgesamt 52 Stunden (42 bis 56 oder 48 bis 56 Stunden). Diese Langzeitstimulation bedeutet den Ausschluss von Sauerstoff von vorzugsweise 52 Stunden. Dies umfasst eine Transportzeit des Blutes für bis zu 24 Stunden unter Ausschluss von Sauerstoff (O_{2d}) und Kohlendioxid (CO₂) gefolgt von weiteren 24h bei 37°C unter O₂ und CO₂ Mangel. Im weiteren Verfahren erfolgt eine Stimulation der Leukozyten (Teil D) für die Dauer von 2 - 8 Stunden, vorzugsweise 4 Stunden. Diese Stimulation umfasst Sauerstoff und Nährstoffmangel.

Der Vorteil des der Erfindung zugrundeliegenden Verfahrens besteht darin, dass das aktivierte Plasma direkt aus dem Blutplasma bzw. Leukozyten des Spenderblutes gewonnen werden kann (autolog) und keine weiteren operativen Eingriffe wie Knochenmarksentnahme oder Fettabsaugung, vorgenommen werden müssen. Dies ist besonders bei Akutpatienten von Vorteil, da eine Operation den Zustand verschlechtern kann. Außerdem ist die Blutabnahme im Vergleich zur Entnahme der MSC mit weniger Kontaminationsrisiken verbunden. Die nach diesem beschriebenen Verfahren generierte pharmazeutisch wirksame Plasmapräparation kann auch alternativ somatisches Immuntherapeutikum genannt werden.

Die durch das Verfahren gewonnene, mit immunmodulatorischen und anti-inflammatorischen Faktoren angereicherte, pharmazeutische Blutplasmagemisch kann dem Patienten im Anschluss vorzugsweise intradermal, aber auch subkutan verabreicht werden. Alternativ kann es auch als Infusion verabreicht werden. Dies kann je nach Indikation einmalig oder wiederholt angewendet werden. Das einmalige Anwendungsvolumen richtet sich nach der Art der Anforderung/Therapie und ob ein Mensch oder ein Tier behandelt werden soll, und kann zwischen 1ml und 500ml liegen. Die Volumina können auch einzeln aliquotiert verabreicht werden.

Das erfindungsgemäße Verfahren stellt dem Patienten schnell ein immunmodulierende/anti-inflammatorisches Immuntherapeutikum bereit, da sie nicht erst wie die Stammzellen für längere Zeit (14 Tage oder länger) in Kultur genommen werden müssen. Daher stellt die vorliegende Erfindung ein Verfahren zur Generierung von anti-entzündlich wirksamen Blutplasma bereit, welches für die schnelle Hilfe für Akutpatienten eingesetzt werden kann. Dies kann beispielsweise für die Behandlung von Patienten mit einer akuten Entzündungsreaktion, wie z.B. der COVID-19 Erkrankung zutreffen.

Erfindungsgemäß wird in diesem Verfahren eine pharmazeutische Präparation mit immunmodulierender/anti-inflammatorischer Wirkung hergestellt und kann daher beispielsweise auch für die Behandlung von Autoimmunerkrankungen wie der Rheumatoiden Arthritis verwendet werden. Hierbei kann das Verfahren wiederholt werden. Beispielsweise kann ein Patient 5 Applikationen zu je 2 × 500 µl subkutan verabreicht bekommen. Beispielsweise in Abständen von 7 Tagen für einen Zeitraum von 35 Tagen.

Zur Behandlung von rheumatoider Arthritis werden Methotrexat und/oder Antikörper gegen pro-inflammatorische Zytokine wie TNF-alpha eingesetzt, die auch schwere Nebenwirkungen hervorrufen können. Hier wäre dieses Verfahren von Vorteil für den Patienten, da Nebenwirkungen im Zusammenhang mit einer Antikörpertherapie oder Standardtherapeutika wie Methotrexat vermieden werden können.

Ferner kann das nach den Ansprüchen hergestellte autologe Immuntherapeutikum auch zur Behandlung von Wundheilungsverzug, Wundheilungsstörungen, chronischen Wunden, insbesondere Ulkus oder Analfisteln, Frostbeulen, Gefäßverengungen, eingesetzt werden.

Außerdem können die immunmodulierenden Eigenschaften des durch das Verfahren bereitgestellten aktivierten Plasmagemisches auch bei Transplantationen (Abstoßung) oder Ischämie zum Einsatz kommen.

Darüber hinaus kann das beschriebene Verfahren auch in der Veterinärmedizin zur Behandlung ähnlicher Erkrankungen oder Verletzungen angewendet werden. Beispielsweise können Pferde mithilfe dieses autologen anti-entzündlichen Plasmagemisches behandelt werden.

Daher kann unter dem Begriff Patient in der vorliegenden Erfindung auch ein Pferd, vorzugsweise Rennpferd oder jedes andere Tier, bei dem man dieses Verfahren durchführen kann, verstanden werden.

### Ausführungsbeispiel:

Einem Arthritis-Patienten werden 50 - 100 ml venöses Blut abgenommen. Die Spritzen oder Monovetten enthalten ein kleines Volumen Heparin als Anti-Koagulanz. Die Spritzen oder Monovetten mit dem Blut werden über Nacht bei einer Raumtemperatur zwischen 15 - 24°C vom Arzt in ein Labor transportiert. Bei diesem Transport beginnt bereits die Sekretion von Zytokinen, hervorgerufen durch die O₂ Mangelversorgung der Zellen in den verschlossenen Spritzen oder Monovetten.

Im Labor angekommen, werden die Volumen aller Spritzen oder Monovetten gepoolt. Das Blut wird zu wesentlichen gleichen Volumen-Teilen (Teil A und B) aufgeteilt und getrennt weiterbearbeitet. Teil A wird für 16 - 24 Stunden in einem Brutschrank bei 35 - 40°C, insbesondere bei 37°C inkubiert.

Nach 16 - 24 Stunden wird das Blut aus den Spritzen/Monovetten pipettiert, gepoolt und abzentrifugiert. Dadurch erfolgt eine Trennung des Plasmas von den restlichen Bestandteilen des Blutes. Das Plasma wird abpipettiert und bis zur weiteren Verwendung aliquotiert und bei -20 bis -180°C kryogelagert, vorzugsweise bei einer Temperatur von -150°C.

Das Blut des Teils B wird mit einem isotonischen Puffer, vorzugsweise PBS ohne Ca++, Mg++ im Verhältnis von 2:1 vermischt. Anschließend wird aus dem PBS/Blut-Gemisch die Fraktion der Leukozyten mittels Dichtegradientenzentrifugation isoliert. Die isolierten Leukozyten werden für 2 - 8 Stunden in einem isotonen Puffer, vorzugsweise 0,9% NaCl, aq bei 18 - 24°C, bevorzugt bei 20°C inkubiert. Es kommt zu einer erneuten Sekretion von Wachstumsfaktoren durch die O₂ und Nährstoff-Mangelversorgung. Die so entstandene isotone Wachstumsfaktoren-Suspension ist das zweite Zwischenprodukt. Bis zur weiteren Verwendung wird dieses zweite Zwischenprodukt aliquotiert und bei -20 bis -180°C kryogelagert, vorzugsweise bei einer Temperatur von -150°C.

Für die Herstellung des applikationsfertigen Endproduktes (Menge E) wird jeweils ein Aliquod des Zwischenproduktes 1 und 2 aufgetaut. Aus beiden Zwischenprodukten wird das Endprodukt hergestellt. Das Mischungsverhältnis kann zwischen einem Anteil von 70 - 95% für das Zwischenprodukt 2 liegen und entsprechend zwischen 5 - 30% für das Zwischenprodukt A.

Das bevorzugte Mischungsverhältnis ist 80% von Zwischenprodukt 2 und 20% von Zwischenprodukt A.

Nach dem beide Zwischenprodukte sorgfältig vermischt wurden, wird dies sterilfiltriert (0,22 oder 0,45µm) und in eine Spritze angesaugt. Das Anwendungsvolumen richtet sich nach der Art des Patienten und der Art der Anforderung/ Therapie und kann einmalig zwischen 1ml und 150ml liegen. Die Applikationen erfolgen z.B. für einen arthritischen Patienten fünfmal subkutan in den Abständen Tag 0, Tag 7, Tag 21, Tag 35 und Tag 49 mit jeweils 2× 500 µl.

Nachfolgend wird die Erfindung anhand der Zeichnung nochmals kurz zusammengefasst. Im Einzelnen zeigt die schematische Darstellung in:

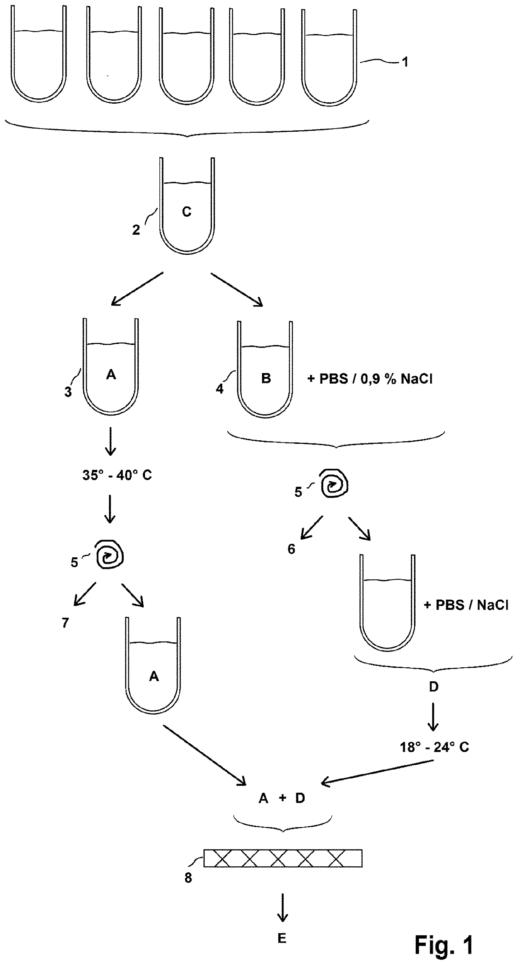

- Fig. 1: eine schematische Darstellung eines erfindungsgemäßen Verfahrens.

Die einzige Fig. zeigt eine schematische Darstellung des Verfahrens nach der Erfindung, wonach ein mit Faktoren und Botenstoffen angereichertem autologen Therapeutikums aus einer Blutspende hergestellt wird, mit den Verfahrensschritten:
- Aufteilen der Menge C des Bluts 1 eines Blutspenders zu zwei Teilen bzw. Teilmengen A und B, wobei die Menge C des Blutes 2 vor dem Aufteilen mit einem Anti-Koagulans versetzt wird, vorzugsweise Heparin oder Citratpuffer.
- Inkubation des Teils A für einen vorgewählten Zeitraum in einem Brutschrank durch thermische Beaufschlagung bei 35° bis 40°C, insbesondere bei 37°C, wobei die Inkubation (thermische Stimulation) der isolierten Leukozyten aus Teil B für 2 bis 8 Stunden in einem isotonen Puffer, vorzugsweise 0,9% NaCl, aq bei 18 - 24°C, bevorzugt bei 20°C, erfolgt, und dass der Teil A des Blutes nach der Inkubation zur Trennung des Plasmas von den restlichen Bestandteilen des Blutes abzentrifugiert 5 wird, wobei die restlichen Bestandteile entfernt werden.
- Abtrennung der Fraktion der Leukozyten durch Isolierung aus Teil B, wobei die Abtrennung der Fraktion der Leukozyten durch Isolierung aus dem Teil B (PBS/Blut-Gemisch) mittels Dichtegradientenzentrifugation 5 oder magnetic bead sorting erfolgt, im Beispiel unter Beimengung von einem isotonischen Puffer (NaCl) zu Teil D. Der Überstand 6 (löslicher bzw. nicht am Boden abgesetzten Teil nach Zentrifugation) wird abgesondert.
- Inkubation des Teils D unter Nährstoffmangel und/oder Sauerstoffmangel im Verhältnis zum normalen Bedarf der Zellen;
- Zusammenführen der Teile A und D zu einer Mischung E, In einem weiteren exemplarischen Schritt erfolgt die Abtrennung der Fraktion der Leukozyten durch Isolierung aus dem Teil B (PBS/ Blut-Gemisch) mittels Dichtegradientenzentrifugation 5 oder magnetic bead sorting.
- in einem letzten Schritt werden aus der Mischung E die Zellen abfiltriert 7.
- Endprodukt E (zur Weiterverarbeitung als Autologes Therapeutikum, Pharmazeutische Zusammensetzung).

### Bezugszeichenliste

- 1: Röhrchen mit Blut nach Blutabnahme
- 2: Röhrchen mit zusammengeführtem (gepooltem) Blut (Teil C)
- 3: Zentrifugenröhrchen gefüllt mit Teil A
- 4: Zentrifugenröhrchen gefüllt mit Teil B
- 5: Zentrifugation
- 6: Überstand (löslicher bzw. nicht am Boden abgesetzten Teil nach Zentrifugation)
- 7: Abtrennung der Nichtplasmafraktion
- 8: Filtration
- A: Teil des Bluts
- B: Teil des Bluts
- C: Menge des Bluts
- D: Teil des Bluts
- E: Endprodukt (Mischung)

## Patentansprüche

1. Verfahren zur Herstellung eines mit Faktoren und Botenstoffen angereichertem autologen Therapeutikums aus einer Blutspende,
**gekennzeichnet durch** die Schritte:
- Aufteilen der Menge C des Bluts eines Blutspenders (1) zu zwei Teilen A und B,
- Inkubation des Teils A für einen vorgewählten Zeitraum in einem Brutschrank **durch** thermische Beaufschlagung bei 35° bis 40°C, insbesondere bei 37°C,
- Abtrennung der Fraktion der Leukozyten **durch** Isolierung aus Teil
B und Beimengung von einem isotonischen Puffer (NaCl) zu Teil D;
- Inkubation des Teils D unter Nährstoffmangel und/oder Sauerstoffmangel im Verhältnis zum normalen Bedarf der Zellen;
- Zusammenführen der Teile A und D zu einer Mischung E.

2. Verfahren nach Anspruch 1,
**dadurch gekennzeichnet,**
**dass** in einem weiteren Schritt aus der Mischung E die Zellen abfiltriert (7) werden.

3. Verfahren nach Anspruch 1 oder 2,
**dadurch gekennzeichnet,**
**dass** die Menge C des Blutes (2) vor dem Aufteilen mit einem Anti-Koagulans versetzt wird, vorzugsweise Heparin oder Citratpuffer.

4. Verfahren nach einem der Ansprüche 1 bis 3,
**dadurch gekennzeichnet,**
**dass** das Blut direkt nach der Blutabnahme mit einer Temperatur unterhalb der normalen Körpertemperatur des Blutspenders über einen vorgewählten Zeitraum von mehreren Stunden gelagert wird.

5. Verfahren nach einem der Ansprüche 1 bis 4,
**dadurch gekennzeichnet,**
**dass** der Teil A des Blutes nach der Inkubation zur Trennung des Plasmas von den restlichen Bestandteilen des Blutes abzentrifugiert (5) wird, wobei die restlichen Bestandteile entfernt werden.

6. Verfahren nach einem der Ansprüche 1 bis 5,
**dadurch gekennzeichnet,**
**dass** die Abtrennung der Fraktion der Leukozyten durch Isolierung aus dem Teil B (PBS/Blut-Gemisch) mittels Dichtegradientenzentrifugation (5) oder magnetic bead sorting erfolgt.

7. Verfahren nach einem der Ansprüche 1 bis 6,
**dadurch gekennzeichnet,**
**dass** die Inkubation der isolierten Leukozyten aus Teil B für 2 bis 8 Stunden in einem isotonen Puffer, vorzugsweise 0,9% NaCl, aq bei 18 bis 24°C, bevorzugt bei 20°C, erfolgt.

8. Verfahren nach einem der Ansprüche 5 bis 7,
**dadurch gekennzeichnet,**
**dass** das Plasma des inkubierten Teils A, insbesondere nach Zentrifugation, bei -20 -180°C kryogelagert wird, vorzugsweise bei einer Temperatur von -150°C und vor der Herstellung des applikationsfertigen Endproduktes aufgetaut wird.

9. Autologes Therapeutikum nach einem der Ansprüche 1 bis 8 zur Verwendung als Medikament.

10. Autologes Therapeutikum nach Anspruch 9 zur Verwendung bei der Behandlung von einer der nachfolgenden Erkrankungen:
- rheumatoider Arthritis, Arthritis,
- Inflammationen (Entzündungen),
- Wundheilungsverzug, Wundheilungsstörungen,
- chronische Wunden, insbesondere Ulkus oder anal Fisteln,
- Frostbeulen, Gefäßverengungen,
- COVID-19 - SARS induzierte Entzündungsreaktionen.

11. Pharmazeutische Zusammensetzung, umfassend ein autologes Therapeutikum nach einem der Ansprüche 9 bis 10 und einen oder mehreren pharmazeutisch annehmbaren Hilfsstoffen.

12. Autologes Therapeutikum nach einem der Ansprüche 9 bis 10 oder pharmazeutische Zusammensetzung nach Anspruch 11 zur Verwendung bei einer Therapie,
oder in einem Verfahren zum Behandeln einer Krankheit oder eines Zustands, wobei das Verfahren ein Verabreichen einer therapeutisch wirksamen Menge des Therapeutikums an ein Subjekt umfasst, wobei die Krankheit oder Zustand ein Entzündungszustand, eine immunregulatorische Störung ist.

13. Autologes Therapeutikum nach Anspruch 9, wobei die Anwendung als Medikament mittels subkutaner oder intradermaler Applikation erfolgt, aber auch als Salbe, Suspension, Spray oder als Aerosol angewendet werden kann.
